(19) Europäisches Patentamt
European Patent Office
Office européen des brevets

(11) Veröffentlichungsnummer: **0 115 295**
**B1**

(12) # EUROPÄISCHE PATENTSCHRIFT

(45) Veröffentlichungstag der Patentschrift: **30.04.86**

(51) Int. Cl.[4]: **C 07 D 475/02**

(21) Anmeldenummer: **84100484.9**

(22) Anmeldetag: **18.01.84**

(54) **Verbessertes Verfahren zur Herstellung von Riboflavin.**

(30) Priorität: **26.01.83 DE 3302497**

(43) Veröffentlichungstag der Anmeldung: **08.08.84 Patentblatt 84/32**

(45) Bekanntmachung des Hinweises auf die Patenterteilung: **30.04.86 Patentblatt 86/18**

(84) Benannte Vertragsstaaten: **CH DE FR GB LI NL**

(56) Entgegenhaltungen:
**US-A-2 807 611**

**CHEMICAL ABSTRACTS, Band 57, 1962, Spalten 11290i-11291a, Columbus, Ohio, USA V.M. BEREZOVSKII et al.: "Catalysts for the reaction of secondary aromatic o-amino azo compounds with trihydroxypyrimidines"**

(73) Patentinhaber: **BASF Aktiengesellschaft, Carl-Bosch-Strasse 38, D-6700 Ludwigshafen (DE)**

(72) Erfinder: **Ernst, Hansgeorg, Dr., Brüsseler Ring 38, D-6700 Ludwigshafen (DE)**
Erfinder: **Schmidt, Wolfram, Dr., Im Rosengarten Ost 8, D-6701 Friedelsheim (DE)**
Erfinder: **Paust, Joachim, Dr., Ringstrasse 3, D-6708 Neuhofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 295 B1

LIBER, STOCKHOLM 1986

**Beschreibung**

Die vorliegende Erfindung betrifft ein verbessertes Verfahren zum Herstellung von Riboflavin (I; Vitamin $B_2$) durch Kondensation eines 4,5-Dimethyl-H-(D)-ribityl-2-phenylazo-anilins (II) mit Barbitursäure (III) in Gegenwart einer Säure als Kondensationsmittel.

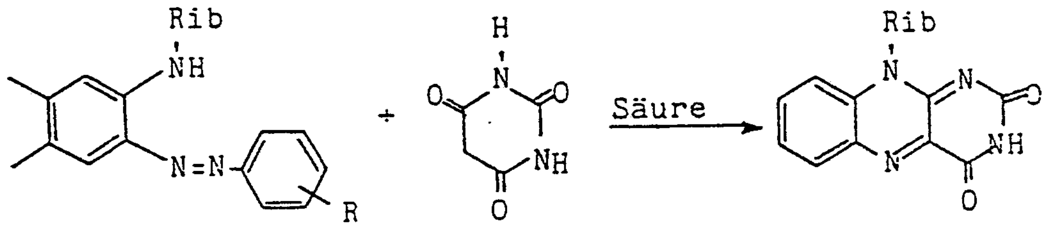


II III I

Rib = Ribityl
R = H; p- oder o-Substituent wie -Cl, $-NO_2$ oder $-CH_3$.

Dieser letzte Schritt der Riboflavin-Synthese ist, sieht man von der er-findungsgemäßen Verbesserung ab, aus mehreren Veröffentlichungen bekannt, beispielsweise aus der Arbeit von Tishler et al (J.Am.Chem.Soc., Band 69 (1947), Seite 1487), nach welcher Eisessig als saures Kondensationsmittel verwendet wurde.

Berezowskii et al (J.Gen.Chem. USSR 1961, Seite 3444) untersuchten außerdem noch eine Reihe von anderen Säuren auf ihre Eignung als Kondensationsmittel. Hiernach erhielt man die besten Ausbeuten an I - etwa 70 % - mit Essigsäure, Phenylessigsäure und Benzoesäure. Allerdings verwendeten sie hierbei einen Barbitursäureüberschuß von 65 mol%. Setzt man II und III dagegen in äquimolaren Mengen ein, so sinkt die Ausbeute an I beträchtlich. So erhielten Haley et al (J.Am.Chem.Soc., Band 76 (1954), S. 2926) bei Verwendung von Eisessig als Kondensationsmittel bei der Umsetzung von II und III im Molverhältnis von 1:1 nur noch eine Ausbeute von 41 % an Riboflavin.

Der Erfindung lag daher die Aufgabe zugrunde, die unbefriedigenden Ausbeuten an I zu verbessern und außerdem die Verwendung molarer Überschüsse an III über II entbehrlich zu machen.

Es wurde nun gefunden, daß man bei der eingangs beschriebenen Kondensationsreaktion deutlich höhere Ausbeuten an Riboflavin erhält, wenn man als saures Kondensationsmittel eine aliphatische oder cycloaliphatisch-aliphatisch tertiäre Carbonsäure der allgemeinen Formel IV verwendet

$$R^2 - \underset{R^3}{\overset{R^1}{C}} - COOH \qquad (IV),$$

in welcher $R^1$, $R^2$ und $R^3$ je für eine niedere Alkylgruppe stehen, wobei $R^1 + R^2 + R^3$ zusammen 3 bis 20, vorzugsweise 3 bis 10 C-Atome enthalten sollen, oder aber $R^1$ für eine niedere Alkylgruppe, insbesondere für eine Methylgruppe, steht und $R^2$ und $R^3$ zusammen für eine Tetramethylengruppe oder eine Pentamethylengruppe stehen.

Als tertiäre Carbonsäuren der allgemeinen Formel IV kommen beispielsweise tertiäre aliphatische Carbonsäuren wie Trimethylessigsäure (Pivalinsäure), 2,2-Dimethyl-butansäure und 2,2-Dimethyl-pentansäure; tertiäre cycloaliphatisch-aliphatische Carbonsäuren, wie 1-Methyl-cyclohexan-1-carbonsäure oder 1-Methylcyclo-pentancarbonsäure oder aber Gemische tertiärer Carbonsäuren in Betracht.

Unter geeigneten Gemischen tertiärer Carbonsäuren verstehen wir insbesondere im Handel erhältliche Gemische synthetischer Säuren, die im wesentlichen gesättigte tertiäre Carbonsäuren der Formel IV enthalten. Genannt seien vor allem Versatic ®-10-Säure, eine synthetische $C_{10}$-Carbonsäure der Shell Chemie sowie ähnliche Produkte der Firma Esso die unter dem Namen "Neosäuren" im Handel sind. Genannt seien hiervon beispielsweise die "Neopentansäure", die als Hauptbestandteil Trimethylessigsäure enthält, und die "Neodecansäure", die in ihrer Zusammensetzung der Versatic-10-Säure ähnlich sein dürfte. Besonders gute Ergebnisse erzielt man mit Trimethylessigsäure oder Versatic® -10-Säure, von denen Versatic®-10-Säure wegen ihrer besseren geruchlichen Eigenschaften sowie wegen ihrer leichteren Handhabung als Flüssigkeit vorzuziehen ist.

Ausgangsverbindungen II sowie deren Herstellung sind bekannt. Im allgemeinen wird man die billigste Verbindung dieser Reihe, nämlich das Phenylazoderivat einsetzen. Jedoch eignen sich prinzipiell auch

Verbindungen, in denen die ortho- oder insbesondere die para-Stellung der Azophenylgruppe durch Substituenten wie Methyl, Chlor oder die Nitrogruppe substituiert sind. Die Ausgangsverbindungen müssen nicht speziell gereinigt werden sondern können auch als Rohprodukte eingesetzt werden. Die Ausbeuteangaben beziehen sich dann auf im Ausgangsmaterial enthaltenes II.

Man nimmt man die Umsetzung, wie auch bisher schon üblich, in Gegenwart eines inerten Verdünnungs- oder Lösungsmittels vor.

Als Lösungsmittel eignen sich vorzugsweise solche, in denen auch das bei der Kondensation entstehende Wasser löslich ist oder zumindest teilweise löslich ist, also Dioxan, Tetrahydrofuran, Dimethylformamid und vor allem die relativ billigen niederen Alkohole mit einem Siedepunkt von 80 bis 150° C, wie Propanol, Isopropanol, n-Butanol, Isobutanol und n-Pentanol, insbesondere Isobutanol.

Die Menge des Lösungsmittels soll zweckmäßigerweise so bemessen sein, daß die Ausgangsverbindungen gerade in Lösung gebracht werden können. Sie liegt im allgemeinen zwischen 2 und 12 1 pro kg II. Nach kurzem Erhitzen beginnt dann das gebildete Riboflavin auszukristallisieren.

Die Menge der Carbonsäure beträgt vorzugsweise 0,5 bis 6 Mol pro Mol II, was beispielsweise für Pivalinsäure etwa 0,19 bis 1,7 kg pro kg II und für Versatic-10-Säure 236 g bis 2,85 kg pro kg II entspricht.

Es ist ein besonderer Vorteil des erfindungsgemäßen Verfahrens, daß man auch bei Einsatz äquimolarer Mengen von II und III deutlich höhere Ausbeuten als bisher erzielt. Diese Ausbeuten betragen etwa 80 %, lassen sich aber noch auf über 90 % erhöhen, wenn man III in einem bis zu 0,15-mola-ren Überschuß verwendet. Höhere Überschüsse stören nicht, erbringen jedoch keine merklichen Ausbeutesteigerungen mehr (vgl. Beispiel 3a und b).

Die Reaktionstemperaturen liegen bei 80 bis 120° C. Vorzugsweise arbeitet man bei Temperaturen über 100° C, also bei etwa 100 bis 115° C sowie in einem entsprechend hoch siedenden Lösungsmittel, vor allem Isobutanol. Bei 100° C beträgt die Reaktionszeit etwa 5 bis 15 Stunden.

Falls man in einem Alkohol wie Isobutanol als Lösungsmittel arbeitet, bieten die erfindungsgemäßen Säuren im Gegensatz zu der bisher vor allem gebräuchlichen Essigsäure den Vorteil, daß sie kaum zur Veresterung neigen, so daß kaum Verluste an Lösungsmittel und Säure entstehen bzw. ein entsprechender Regenerierungsaufwand entfällt.

Die Aufarbeitung des Reaktionsgemisches kann nach den üblichen Methoden erfolgen, etwa indem man es abkühlen läßt und das auskristallisierte Riboflavin abfiltriert.

**Beispiele 1 und 2 sowie Vergleichsbeispiele 1 bis 7**

Ein Gemisch aus 40 g (0,111 mol) 4,5-Dimethyl-N-(D)-Ribityl-2-phenylazo-anilin, 14,2 g (0,111 mol) Barbitursäure, 30 g (0,295 mol) Pivalinsäure in 190 ml Isobutanol wurde 10 Stunden lang unter Rückflußkühlung bei 105 bis 110° C gerührt und danach auf Raumtemperatur (RT) abgekühlt. Der gebildete Riboflavinniederschlag wurde abfiltriert, mit Methanol sowie kaltem und heißem Wasser gewaschen und unter vermindertem Druck bei 60° C getrocknet.

Zum Vergleich wurde die Reaktion unter sonst gleichen Bedingungen mit jeweils 0,295 Mol einiger anderer Säuren als Kondensationsmittel wiederholt. Die Ergebnisse sind in der folgenden Tabelle dargestellt. Die Reinheit des Riboflavins wurde durch UV-Messung gemäß Pharmakopoe Europa bestimmt.

**Tabelle**

| Beispiele | Kondensations-mittel | Riboflavin-Ausbeute [%] | Reinheit [%] | Reinaus-beute [%] |
|---|---|---|---|---|
| a) erfindungs-gemäß | | | | |
| 1 | Pivalinsäure | 78,6 | 98,2 | 77,2 |
| 2 | Versatic-10-Säure | 79,9 | 96,5 | 77,1 |
| b) zum Vergleich | | | | |
| 1 | Essigsäure | 74,2 | 94,7 | 70,3 |
| 2 | Propionsäure | 73,9 | 95,5 | 70,6 |
| 3 | n-Buttersäure | 75,8 | 93,6 | 70,9 |
| 4 | Isobuttersäure | 74,6 | 93,2 | 69,5 |
| 5 | n-Valeriansäure | 73,9 | 94,6 | 69,9 |
| 6 | Isovaleriansäure | 75,9 | 92,3 | 70,0 |
| 7 | Hexansäure | 73,4 | 93,4 | 68,6 |

**Beispiel 3**

a) Arbeitet man unter den Bedingungen von Beispiel 1, verwendet jedoch anstelle von 14,2 g (0,111 mol) Barbitursäure einen 11 %igen Über-schuß, d.h. 15,8 g (0,123 mol), so erhält man 36,5 g Riboflavin (87,5 % d.Th.) mit einer Reinheit von 96,2 % (nach UV).

b) Arbeitet man unter den Bedingungen von Beispiel 1, verwendet jedoch einen 26 %igen Überschuß an Barbitursäure, d.h. 17,9 g (0,14 mol) an Barbitursäure, so erhält man 36,2 g (86,8 % d.Th.) Riboflavin mit einer Reinheit von 95,5 %.

**Beispiel 4**

36 g (0,1 mol) 4,5-Dimethyl-N-(D)-Ribityl-2-phenylazoanilin und 14,2 g (0,111 mol) Barbitursäure wurden in einem Gemisch aus 170 ml iso-Butanol und 73,5 ml (0,393 mol) Versatic-10-Säure 10 h unter Rückfluß gerührt. Anschliessend ließ man das Reaktionsgemisch auf RT abkühlen, filtrierte ab, wusch den Niederschlag mit Methanol, dann mit kaltem und heißem Wasser und trocknete ihn unter vermindertem Druck bei +60°C. Man erhielt 34,9 g Riboflavin (92,8 % d.Th.) mit einer Reinheit von 95,6 % (best. mit UV).

**Beispiel 5**

40 g (0 111 mol) 4,5-Dimethyl-N-(D)-Ribityl-2-phenylazoanilin und 15,8 g (0,123 mol) Barbitursäure wurden in einer Mischung aus 190 ml iso-Butanol mit 80 ml (0,428 mol) Versatic-10-Säure 10 h unter Rückfluß gerührt. Anschließend ließ man das Reaktionsgemisch auf RT abkühlen, wusch den Niederschlag mit Methanol, kaltem und heißem Wasser und trocknete unter vermindertem Druck bei +60°C. Man erhielt 38,8 g Riboflavin (93 % d.Th.) mit einer Reinheit von 95,6 %.

**Beispiel 6**

36 g (0,1 mol) 4,5-Dimethyl-N-(D)-Ribityl-2-phenylazoanilin und 14,85 g (0,116 mol) Barbitursäure wurden in einem Gemisch aus 170 ml iso-Butanol und 60 ml (0,532 mol) Pivalinsäure 6 h unter Rückfluß zum Sieden erhitzt. Nach Aufarbeitung wie in Beispiel 4 erhielt man 33,1 g Riboflavin (88,0 % d.Th.) mit einer Reinheit von 98,3 %.

**Beispiel 7**

36 g (0,1 mol) 4,5-Dimethyl-N-(D)-Ribityl-2-phenylazoanilin und 14,2 g (0,111 mol) Barbitursäure wurden in einem Gemisch aus 170 ml n-Pentanol und 30 ml (0,266 mol) Pivalinsäure 10 h bei 115°C gerührt. Nach Abkühlen auf RT wurde gemäß Beispiel 4 aufgearbeitet. Man erhielt 32,9 g Riboflavin (87,5 % d.Th.) einer Reinheit von 95,5 %.

**Beispiel 8**

40 g (0,111 mol) 4,5-Dimethyl-N-(D)-Ribityl-2-phenylazoanilin und 15,8 g (0,123 mol) Barbitursäure wurden in einem Gemisch aus 120 ml Isobutanol und 98 ml (0,532 mol) Versatic-10-Säure 6 h unter Rückflußbedingungen gerührt. Nach Aufarbeitung wie in Beispiel 4 erhielt man 39,6 g Riboflavin (94,9 % d.Th.) mit einer Reinheit von 93,4 %.

**Beispiel 9**

33,6 g (93,6 mmol) 4,5-Dimethyl-N-(D)-Ribityl-2-phenylazoanilin und 13 7 g (107 mmol) Barbitursäure wurden

in einem Cemisch aus 170 ml Isobutanol und 98 ml Neodekansäure ® (Esso) 10 h unter Rückflußbedingungengerührt. Anschließend ließt man das Reaktionsgemisch auf RT abkühlen, wusch den Niederschlag mit Methanol, kaltem und heißem Wasser und trocknete ihn unter vermindertem Druck bei +70°C. Man erhielt 33,4 g Riboflavin (94,9 %) mit einer Reinheit von 94 % (bestimmt nach Ph. Eur.)

**Beispiel 10**

36,0 g (0,1 mol) 4,5-Dimethyl-N-(D)-Ribityl-2-phenylazoanilin und 14,7 g (0,115 mol) Barbitursäure wurden in einem Gemisch aus 120 ml Dioxan und 98 ml Versatic-10-Säure 10 h unter Rückflußbedingungen gerührt. Nach Aufarbeitung analog Beispiel 4 erhielt man 37,7 g Riboflavin (Rohausbeute quantitativ) mit einer Reinheit von 96 %.

**Beispiel 11**

36,0 g (0,1 mol) 4,5-Dimethyl-N-(D)-Ribityl-2-phenylazoanilin und 14,7 g (0,115 mol) Barbitursäure wurden in einem Gemisch aus 170 ml Dioxan und 60 ml Pivalinsäure 10 h unter Rückflußbedingungen gerührt. Nach Aufarbeitung analog Beispiel 4 erhielt man 36,5 g (97,0 %) Riboflavin mit einer Reinheit (Ph. Eur.) von 97 %.

**Beispiel 12**

33,6 g (93,6 mmol) 4,5-Dimethyl-N-(D)-Ribityl-2-phenylazoanilin und 13 7 g (107 mmol) Barbitursäure wurden in einem Gemisch aus 120 ml 1,2-Di-methoxyethan und 105 ml Versatic-10-Säure 14 h unter Rückflußbedingungen gerührt. Nach Aufarbeitung analog Beispiel 4 erhielt man 33,5 g Riboflavin (95,2 %) mit einer Reinheit von 95 % (nach Ph. Eur.)

**Patentansprüche**

Verfahren zur Herstellung von Riboflavin der Formel I

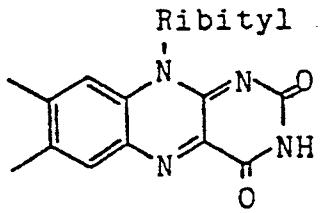

(I)

durch Kondensation eines 4,5-Dimethyl-N-(D)-ribityl-2-phenylazo-anilins der Formel II

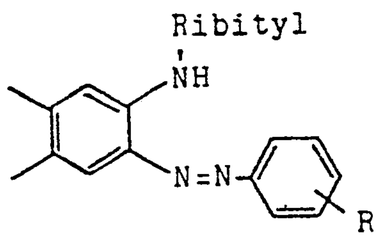

(II),

in der R H oder -Cl, $-NO_2$ oder $-CH_3$ in o- oder p-Stellung bedeutet
mit Barbitursäure der Formel III

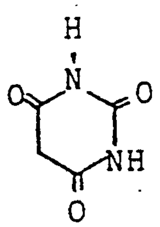

(III)

in Gegenwart einer Säure als Kondensationsmittel und eines inerten Lösungsmittels, dadurch gekennzeichnet, daß man als saures Kondensationsmittel eine aliphatische oder cycloaliphatisch-aliphatische tertiäre Carbonsäure der allgemeinen Formel IV verwendet

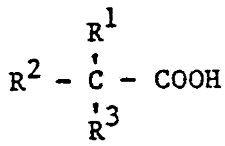

(IV),

in welcher $R^1$, $R^2$ und $R^3$ je für eine niedere Alkylgruppe stehen, wobei $R^1+R^2+R^3$ zusammen 3 bis 20, vorzugsweise 3 bis 10 C-Atome, enthalten sollen, oder aber $R^1$ für eine miedere Alkylgruppe, insbesondere fpü eine Methylgruppe, steht und $R^2$ und $R^3$ zusammen für eine Tetramethylengruppe oder eine Pentamethylengruppe stehen.

2. Verfahren zur Herstellung von Riboflavin gemäß Anspruch 1, dadurch gekennzeichnet, daß man als saures Kondensationsmittel Trimethylessigsäure verwendet.

3. Verfahren zur Herstellung von Riboflavin gemäß Anspruch 1, dadurch gekennzeichnet, daß man als saures Kondensationsmittel Versatic®-10-Säure verwendet.

4. Verbessertes Verfahren zur Herstellung von Riboflavin gemäß Amspruch 1, dadurch gekennzeichnet, daß man das 4,5-Dimethyl-N-(D)-ribityl-2-phenylazo-anilin der Formel II und die Barbitursäure 111 in einem Molverhältnis 11:111 von 1:1 bis 1:1,15 einsetzt.

5. Verbessertes Verfahren zur Herstellung von Riboflavin gemäß Anspruch 1, dadurch gekennzeichnet, daß man die Kondensation in Isobutanol als Lösungsmittel ausführt.

## Claims

1. A process for the preparation of riboflavin of the formula I

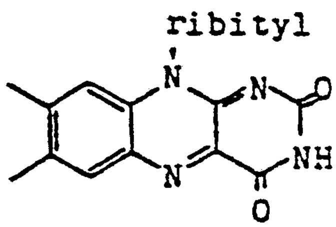

(I)

by condensing a 4,5-dimethyl-N-(D)-ribityl-2-phenylazo-aniline of the formula II

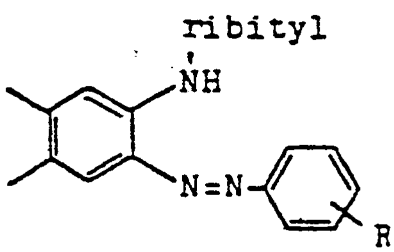

(II),

where R is H or -Cl, $-NO_2$ or $-CH_3$ in the o- or p-position, with barbituric acid of the formula III

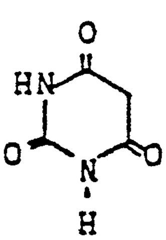

(III)

in the presence of an acid as condensing agent, and of an inert solvent, wherein the acidic condensing agent used is an aliphatic or cycloaliphatic/aliphatic tertiary carboxylic acid of the general formula IV

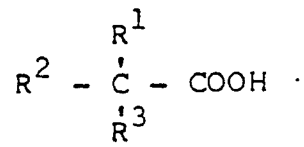 (IV),

where $R^1$, $R^2$ and $R^3$ are each a lower alkyl group, $R^1$, $R^2$ and $R^3$ together containing 3 to 20, preferably 3 to 10, carbon atoms or $R^1$ is a lower alkyl group in particular methyl, and $R^2$ and $R^3$ together form a tetramethylene or pentamethylene group.

2. A process for the preparation of riboflavin as claimed in claim 1, wherein the acidic condensing agent used is trimethylacetic acid.

3. A process for the preparation of riboflavin as claimed in claim 1, wherein the acidic condensing agent used is Versatic® 10-acid.

4. An improved process for the preparation of riboflavin as claimed in claim 1, wherein the molar ratio of the 4,5-dimethyl-N-(D)-ribityl-2-phenylazoaniline of the formula II to barbituric acid III is from 1:1 to 1:1.15.

5. An improved process for the preparation of riboflavin as claimed in claim 1, wherein the condensation is carried out in isobutanol as the solvent.

**Revendications**

1. Procédé de préparation de riboflavine de formule I

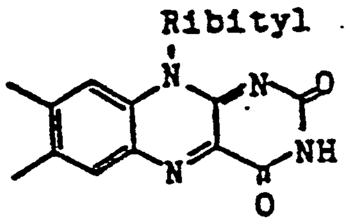 (I)

par condensation d'une 4,5-diméthyl-N-(D)-ribityl-2-phénylazo-aniline de formule II

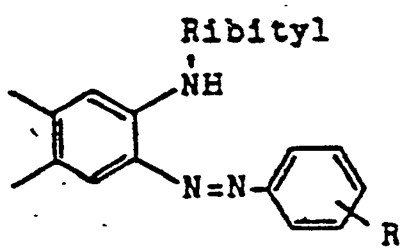 (II),

dans laquelle R représente R ou -Cl, $-NO_2$ ou $-CR_3$ en position o ou p, avec un acide barbiturique de formule III

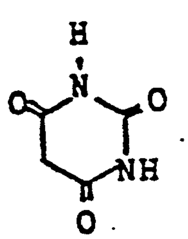 (III)

en présence d'un acide servant d'agent de condensation et en présence d'un solvant inerte, caractérisé en ce qu'on utilise, en tant qu'agent acide de condensation, un acide carboxylique tertiaire aliphatique ou cycloaliphatique-aliphatique de formule générale IV

$$R^2 - \overset{\displaystyle R^1}{\underset{\displaystyle R^3}{\overset{|}{\underset{|}{C}}}} - COOH \qquad (IV)$$

dans laquelle $R^1$, $R^2$ et $R^3$ sont mis chacun pour un groupe alkyle inférieur, $R^1 + R^2 + R^3$ devant contenir au total 3 à 20, de préférence 3 à 10 atomes de C, ou bien $R^1$ est mis pour un groupe alkyle inférieur, en particulier pour un groupe méthyle, et $R^2$ et $R^3$ étant mis ensemble pour un groupe tétraméthylene ou pour un groupe pentaméthylène.

2. Procédé de préparation de riboflavine selon la revendication 1, caractérisé en ce qu'on utilise, en tant qu'agent acide de condensation, l'acide triméthylacétique.

3. Procédé de préparation de riboflavine selon la revendication 1, caractérisé en ce qu'on utilise, en tant qu'agent acide de condensation, l'acide Versatic®-10

4. Procédé amélioré de préparation de riboflavine selon la revendication 1, caractérisé en ce qu'on met en réaction la 4,5-diméthyl-N-(D)-ribityl-2-phénylazo-aniline de formule II et l'acide barbiturique III dans un rapport molaire II : III de 1 : 1 à 1 : 1,15.

5. Procédé amélioré de préparation de riboflavine selon la revendication 1, caractérisé en ce qu'on mène la condensation dans l'isobutanol servant de solvant.